(19) Europäisches Patentamt
European Patent Office
Office européen des brevets

(11) EP 0 827 958 A1

(12) EUROPÄISCHE PATENTANMELDUNG

(43) Veröffentlichungstag:
11.03.1998 Patentblatt 1998/11

(51) Int. Cl.[6]: C07D 307/66

(21) Anmeldenummer: 97115109.7

(22) Anmeldetag: 01.09.1997

(84) Benannte Vertragsstaaten:
AT BE CH DE DK ES FI FR GB GR IE IT LI LU MC NL PT SE
Benannte Erstreckungsstaaten:
AL LT LV RO SI

(30) Priorität: 04.09.1996 CH 2178/96

(71) Anmelder: LONZA AG
CH-3945 Gampel/Wallis (CH)

(72) Erfinder: Schröer, Josef, Dr.
3904 Naters (Kanton Wallis) (CH)

(74) Vertreter:
KUHNEN, WACKER & PARTNER
Alois-Steinecker-Strasse 22
85354 Freising (DE)

# (54) Verfahren zur Herstellung von Arylazotetronsäurederivaten

(57) Es wird ein neues Verfahren zur Herstellung von Arylazotetronsäurederivaten der allgemeinen Formel

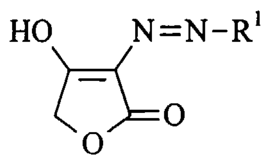

I

beschrieben, worin ein 3-Chlor-4-hydroxy-2(5H)-furanon der Formel

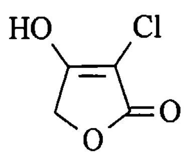

II

diazotiert wird.
Die Arylazotetronsäurederivate sind wichtige Zwischenprodukte zur Herstellung von (+)-Biotin.

EP 0 827 958 A1

## Beschreibung

Die Erfindung betrifft ein neues Verfahren zur Herstellung von Arylazotetronsäurederivaten der allgemeinen Formel

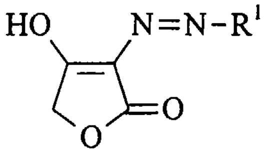

I

worin $R^1$ eine gegebenenfalls substituierte Phenylgruppe bedeutet.

Die Arylazotetronsäurederivate sind wichtige Zwischenprodukte für die Herstellung von (+)-Biotin.

Das ebenfalls als Vitamin H bekannte (+)-Biotin wird u.a. für pharmazeutische Anwendungen und auch als Zusatz in Futtermitteln verwendet.

Für die Herstellung von (+)-Biotin sind zahlreiche Synthesen veröffentlicht worden. Kommerzielle Bedeutung erlangt hat z.B. die in der EP-A 0 270 076 und in der EP-A 273 270 veröffentlichte Totalsynthese von (+)-Biotin ausgehend von Tetronsäure. Die Tetronsäure als Ausgangsprodukt dieser Totalsynthese konnte gemäss EP-A 0 153 615 ausgehend von einem 4-Chloracetessigsäureester durch Chlorierung mit Sulfurylchlorid zum 2,4-Dichloracetessigester, durch thermische Behandlung und Ringschluss zur 3-Chlortetronsäure und schliesslich durch Hydrierung der Chlortetronsäure zur Verfügung gestellt werden. Erst darauf konnte gemäss der genannten EP-A 0 270 076 in einem weiteren, nunmehr vierten Schritt die Diazotierung ansetzen.

Es ist offensichtlich, dass die genannte Bereitstellung der Ausgangsprodukte aufwendig ist. Die Aufgabe bestand folglich darin ein Verfahren zu entwickeln, das diesen Nachteil nicht beinhaltet.

Gelöst werden konnte diese Aufgabe mit dem neuen Verfahren gemäss Patentanspruch 1.

Gemäss dem erfindungsgemässen Verfahren wird 3-Chlor-4-hydroxy-2(5H)-furanon der Formel

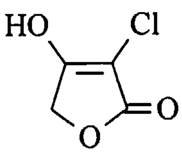

II

mit einem Diazoniumsalz der allgemeinen Formel

$$R^1N_2^{\oplus}X^{\ominus} \qquad \text{III}$$

worin $R^1$ die genannte Bedeutung hat und X ein Säureanion bedeutet, umgesetzt 3-Chlor-4-hydroxy-2(5H)-furanon kann wie vorstehend beschrieben gemäss der EP-A 0 153 615 aus dem 4-Chloracetessigsäureester hergestellt werden.

Die Bereitstellung des Diazoniumsalzes erfolgt auf seit langem bekannte Art und Weise (vgl. EP-A 0 270 076) durch Umsetzung eines entsprechenden Anilins mit einer wässrigen Mineralsäure in Gegenwart eines Alkalinitrites.

Bevorzugt hat $R^1$ die Bedeutung einer Phenylgruppe. Als allfällige Substituenten der Phenylgruppe können auftreten: $C_{1-6}$-Alkyl d.h. zweckmässig Methyl, Ethyl, n-Propyl, iso-Propyl, n-Butyl, sec.-Butyl, tert.-Butyl, Pentyl oder Hexyl und seine Isomeren, $C_{1-6}$-Alkoxy, Aryl d.h. zweckmässig Phenyl oder Aryloxy wie z. B. Phenoxy.

X als Säureanion hat zweckmässig die Bedeutung eines Anions einer Mineralsäure d.h. eines Halogenatoms wie z. B. Fluor, Chlor, Brom oder Jod oder von Hydrogensulfat oder $BF_4$.

Bevorzugtes Diazoniumsalz ist das aus Anilin und Salzsäure hergestellte Benzoldiazoniumchlorid, worin $R^1$ Phenyl und X Chlor bedeutet.

Vorteilhaft wird das Diazoniumsalzes zu einer auf einen pH-Wert von 2 bis 7 gestellten wässrigen Lösung von 3-Chlor-4-hydroxy-2(5H)-furanon, die zweckmässig eine Temperatur von -20 °C bis 20 °C aufweist, wurde zugegeben.

Die Diazotierung erfolgt bevorzugt bei einer Temperatur von -10 °C bis 10 °C.

Das resultierende Arylazotetronsäurederivat fällt in der Regel aus und kann damit auf einfache Weise aus dem Reaktionsgemisch bereits in sehr guter Ausbeute und Reinheit gewonnen werden.

**Beispiel:**

Bei -10°C wurde zu 7 ml halbkonzentrierter wässriger Salzsäure (ca. 16% ig) zuerst tropfenweise 1,4 ml Anilin (15,4 mmol) und anschliessend eine Lösung von 1,0556 g Natriumnitrit (15,3 mmol) in 3 ml Wasser gegeben. Nach 10 min wurde diese blassgelbe, klare Lösung bei gleicher Temperatur mit einer gesättigten wässrigen Natriumacetatlösung titriert, bis ein Farbumschlag nach intensivgelb hin zu beobachten war. Gleichzeitig wurde die Lösung trübe.
Eine Suspension von 3-Chlor-4-hydroxy-2(*5H*)-furanon in 20 ml Wasser wurde mit 1M wässriger Natronlauge auf ca. pH 6 eingestellt, wobei eine klare Lösung erhalten wurde. Diese Lösung wurde auf -10°C gekühlt.
Anschliessend wurde das zuvor preparierte Diazoniumsalz-Reagenz tropfenweise zugegeben. Ein dicker gelboranger Niederschlag fiel bei dieser Operation aus. Das Kühlbad wurde entfernt und man liess auf Raumtemperatur erwärmen. 2,4 g Natriumthiosulfat (15,2 mmol) wurden als Feststoff zugegeben, man rührte 5 min und filtrierte. Der Niederschlag wurde mit 50 ml Wasser gewaschen. Im Filtrat war die Abscheidung von weiterem Produkt zu beobachten, welches ebenfalls durch Filtration gewonnen wurde. Beide Produktfraktionen wurden im Vakuum bei 50°C 18 Stunden lang getrocknet.
Es wurden insgesamt 1,2232 g 3-Phenylazotetronsäure erhalten. Ausbeute: 39%.

| | |
|---|---|
| $^1$H-NMR ($CDCl_3$, 400 Mhz)$\delta$: | 4,50 (d, 2H) |
| | 7,36 (m, 1H) |
| | 7,48 (m, 2H) |
| | 7,58 (m, 2H) |
| | 13,12 (br,s, 0,5H) |
| | 14,00 (br,s, 0,5H) |

**Patentansprüche**

1. Verfahren zur Herstellung von einem Arylazotetronsäurederivat der allgemeinen Formel

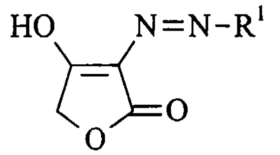

I

worin $R^1$ eine gegebenenfalls substituierte Phenylgruppe bedeutet, dadurch gekennzeichnet, dass 3-Chlor-4-hydroxy-2(5H)-furanon der Formel

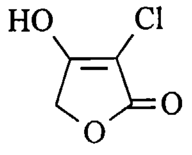

II

mit einem Diazoniumsalz der allgemeine Formel

$$R^1N_2^{\oplus}X^{\ominus} \quad \text{III}$$

worin $R^1$ die genannte Bedeutung hat und X ein Säureanion bedeutet, in das Endprodukt überführt wird.

2. Verfahren gemäss Patentanspruch 1 dadurch gekennzeichnet, dass die Diazotierung bei einer Temperatur von -20 °C bis 20 °C durchgeführt wird.

3. Verfahren gemäss Patentanspruch 1 oder 2 dadurch gekennzeichnet, dass die Zugabe des Diazoniumsalzes zu einer auf einen pH-Wert von 2 bis 7 gestellten wässrigen Lösung von 3-Chlor-4-hydroxy-2(5H)-furanon erfolgt.

Europäisches Patentamt

# EUROPÄISCHER RECHERCHENBERICHT

Nummer der Anmeldung
EP 97 11 5109

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
| A | K. TANAKA ET AL.: "Syntheses and Antimicrobial Activities of Five-Membered Heterocyles Having a Phenylazo Substituent" CHEMICAL AND PHARMACEUTICAL BULLETIN., Bd. 32, Nr. 8, 1984, TOKYO JP, Seiten 3291-3298, XP002049371 * Seite 3292; Beispiele 2A,2I; Tabelle I * * Zusammenfassung * * Seite 3296 * --- | 1-3 | C07D307/66 |
| D,A | EP 0 270 076 A (LONZA AG) * Seite 10, Schema 1 * * Seite 18 - Seite 19; Beispiel 1 * --- | 1-3 | |
| D,A | EP 0 153 615 A (LONZA AG) * das ganze Dokument * ----- | 1-3 | |
| | | | RECHERCHIERTE SACHGEBIETE (Int.Cl.6) |
| | | | C07D |

Der vorliegende Recherchenbericht wurde für alle Patentansprüche erstellt

| Recherchenort | Abschlußdatum der Recherche | Prüfer |
|---|---|---|
| DEN HAAG | 5.Dezember 1997 | Paisdor, B |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie,übereinstimmendes Dokument

EPO FORM 1503 03.82 (P04C03)